# EUROPEAN PATENT APPLICATION

(11) **EP 3 366 650 A1**
(43) Date of publication of application: **29.08.2018**
(21) Application number: 18158364.2
(22) Date of filing: 23.02.2018
(51) Int. Cl.: C02F 1/28, C02F 1/68, C02F 101/34, C02F 101/38, C02F 103/42, C07D 251/32, C02F 101/18

(54) **PROCEDURE AND SYSTEM FOR THE EXTRACTION OF ISOCYANURIC ACID IN SOLUTION**

(30) Priority: 27.02.2017 ES 201730257
(71) Applicant: Diasa Industrial, S.A., 26500 Calahorra (ES)
(72) Inventor: MACÍAS GÁLLEGO, CARLOS, 14006 CORDOBA (ES)
(74) Representative: Maslanka Kubik, Dorota Irena

(57) **Abstract**

A procedure and a system for the extraction of isocyanuric acid is disclosed. According to the invention, the water containing isocyanuric acid is passed through a bed with an adsorbent material, the bed regeneration is carried out by using a liquid regeneration medium, the liquid regeneration medium that contains isocyanuric acid in solution is collected, its recovery is carried out by adding a solid complexing substance, the suspension obtained is passed through a filtering medium and isocyanuric acid is recovered and the liquid extraction medium is regenerated. The system comprises an adsorption column, a deposit of regenerating substance, a filtering medium, a dosage tank to dose the regenerating substance, a compressor, a drive pump and a valves system to configure the equipment according to the phase of the cycle in which it has to operate.

## Description

### Field of the invention

This invention is related generally to the field of the control of the isocyanuric acid level in pool water, and more particularly, to a procedure and a system for the extraction of isocyanuric acid, keeping it within safe levels for the disinfecting action of chlorine.

### Backgrounds of the invention

The addition of chlorine in the form of trichloroisocyanuric acid is widely spread in the world of swimming pools because it is the method that achieves a more effective action of chlorine due to its slow and persistent release over time. However, the addition of chlorine in this form ends up causing the accumulation of isocyanuric acid in water and from certain levels it starts to inhibit the disinfecting action of chlorine by recombination.

Currently, the method usually carried out to control the concentration of isocyanuric acid in water is by means of the substitution of the corresponding volume of water for new water, what supposes a huge waste of this scarce resource.

Some procedures have been tested for the removal of isocyanuric acid from water but none of them seems to be viable on an industrial scale itself.

A procedure known in the prior technique is the method, according to the patent application US2011259835, which is used to reduce the concentration of isocyanuric acid level in the pool water to prevent from blockage of chlorine. The method comprises the stages of adding an effective amount of sodium hydroxide combined with an antimicrobial agent for the pool water and reacting the sodium hydroxide with the isocyanuric acid within it so as to reduce the concentration of isocyanuric acid. The antimicrobial agent reduces the amount of microorganisms which have accumulated in the pool water until chlorine is released.

From the document US5194162 it is known a method to reduce the concentration of isocyanuric acid in the pool water to prevent from blockage of chlorine. The method comprises the stages of adding an effective amount of sodium hydroxide to the pool water and reacting the sodium hydroxide with the isocyanuric acid within it so as to reduce the concentration of isocyanuric acid to a level of between about 40 to 60 PPM.

Another procedure known in the prior technique is the adsorption in activated carbon. The isocyanuric acid is well adsorbed in this material when the contaminated water is passed through a granular bed in the proper dynamic conditions; however, once the carbon is saturated its replacement is necessary so, unless a method of regeneration is available in situ, the procedure is too laborious and expensive.

Another system known of the prior technique is the complexation of isocyanuric acid with melamine so as to form melamine isocyanurate which is in the form of microscopic crystals suspended in water. Nevertheless, this system has several drawbacks: on one hand, in the conditions of the pool water, the amount of melamine which reacts with cyanuric is low, so a high amount of melamine must be dosed so as to complex a significant amount of cyanuric, leading to problems of turbidity and toxicity in the pool, since the combination of cyanuric and melamine is toxic. This mixture will remain in the pool constantly since melamine is soluble in water at a rate of 3100 mg/L, what also may lead to problems of coloring of water. On the other hand, the crystals formed must be removed from the water, what is not feasible with the filtering systems usually used in pools because they are excessively thin and deformable.

Likewise, it still remains in the technique the need for a procedure and a system which allows isocyanuric acid to be extracted from the water in an effective and viable way on an industrial scale, keeping it within safe levels for the disinfecting action of chlorine and, also, recovering it for later use in the manufacture of other chemical components, such as for the manufacture of trichloroisocyanuric acid for the sterilization of swimming pools due to its high percentage of free chlorine. On the other hand, it would be desirable to have a procedure and a system with a closed and ecological circuit.

### Summary of the invention

In order to solve the problems of the prior technique, in a first aspect this invention discloses a procedure for the extraction of isocyanuric acid in solution which comprises the following stages:
(a) In a first step, being this the process of adsorption, the water that contains isocyanuric acid is passed through a bed with an adsorbent material,
(b) The water that passes through the bed is constantly returned to the source of origin so that the concentration of isocyanuric acid decreases with time,
(c) Once the isocyanuric acid level determined according to the needs has been reached, that is, at safe levels for the disinfecting action of chlorine in the source of origin, the second step is carried out, the regeneration of the bed by using a liquid medium of regeneration, being this a polar solvent which has a bigger affinity for the isocyanuric acid than that of the adsorbent,
(d) It is collected the liquid medium of regeneration which contains a concentration of isocyanuric acid in solution and whose recovery is carried out in the third step of the procedure, by the addition of a complexing solid substance of isocyanuric acid,
(e) A suspension of crystals is obtained,
(f) The mixture obtained in the stage(e), in the third step, is passed through a filtering medium of adequate mesh size for the retention of the solid mixture obtained,
(g) The solid mixture of isocyanurate is recovered, and the liquid extraction medium regenerated,
(h) The regenerated liquid extraction medium is returned to the system for the next cycle of the bed regeneration.

The combination of the stages and means used ensures a single procedure which eliminates the drawbacks of the prior technique, providing advantages for the client at the same time.

The three steps of the procedure, that is, the step of adsorption, regeneration and filtration are repeated cyclically, allowing to control the isocyanuric acid level in pool water. With this invention, the excess of dissolved isocyanuric acid is extracted from the water and recovered later in a solid mixture with other components. This mixture can also be used as raw material for the manufacture of trichloroisocyanuric acid, thus, recycling this compound instead of being removed to the environment by the dumping of water. It should be noted that there is no significant waste of water or other reagents used in the process, what is an important indicator when assessing the usefulness and application of the procedure.

Briefly, the procedure according to this invention consists of passing the aqueous solution of isocyanuric acid through an adsorbent bed, extracting the compound from the adsorbent bed by a liquid substance of higher affinity for the compound than the adsorbent and, finally, recovering the compound concentrated in the liquid substance by the addition of a solid substance in powder which complexes the isocyanuric acid completely and it is separated from the liquid substance by means of an adequate filtration system.

According to another aspect, this invention discloses a system for the extraction of isocyanuric acid in solution which comprises:
- a pump, which, in the adsorption cycle, drives the water from the source of origin through flow regulation means to an adsorption column where the isocyanuric acid is retained,
- flow regulation means that allow the isocyanuric acid free water to return to the source of origin,
- a deposit for liquid regeneration medium, connected to a dosage tank which is added a solid complexing substance, optionally together with a filtering adjuvant,
- flow regulation means that allow the liquid regeneration medium to pass from the deposit for the liquid regeneration medium to the dosage tank through other flow regulation means,
- a pump that drives the regeneration liquid coming from the deposit for liquid regeneration medium through u flow regulation means to the dosage tank through other flow regulation means,
- a solid mixture retention filter,
- a dosing pump which, in the regeneration cycle, doses in line the suspension from the dosage tank towards the retention filter,
- flow regulation means that allow the supply of the liquid medium coming from the dosage tank to the retention filter,
- flow regulation means that allow to supply the regeneration liquid recovered from the retention filter to the adsorption column in the opposite direction to the adsorption cycle,
- flow regulation means that return the regeneration liquid after passing through the adsorption column to the deposit for the liquid regeneration medium.

The use of the system according to this invention allows to decrease the isocyanuric acid level in the source of origin and, at the same time, it allows to obtain raw material for the manufacture of trichloroisocyanuric acid. The invention provides a closed circuit, thus, there is no waste of chemical means. At the same time, the system is easy to implement and commercially viable.

### Brief description of the figures

This invention will be better understood with reference to the following figures which illustrate a preferred embodiment of the invention, provided as an example, and which should not be interpreted as limiting of the invention in any way.
Figure 1 is a view of the system according to the first preferred embodiment of this invention.
Figure 2 is a view of the system according to the second preferred embodiment of this invention.

### Detailed description of the preferred embodiments

Below it is provided a detailed description of this invention.

The object of this invention is a general procedure for the extraction and recovery of isocyanuric acid from a aqueous solution. This new procedure is applicable mainly to the level control of this compound in pool water, keeping it within safe levels for the disinfecting action of chlorine.

The procedure for the extraction of isocyanuric acid in solution according to this invention comprises the following stages:
In the stage (a), being this the first step of the procedure called adsorption process, the water containing isocyanuric acid is passed through a bed with a adsorbent material. The adsorbent material used in this stage is selected from the group of materials in the form of porous carbon which has a high affinity for isocyanuric acid, it is preferably selected from the group which comprises activated carbon, carbon airgel, carbon xerogel, activated carbon fibers, ordered mesoporous carbon (OMC) or graphene gels. In a preferred embodiment of this invention, activated carbon is used.

In the stage (b), the water passing through the bed is constantly returned to the source of origin, being this usually a swimming pool, so that the concentration of isocyanuric acid decreases with time.

In the stage (c), once the determined isocyanuric acid level has been reached, being the adequate levels according to the current guidelines between 30 and 50 ppm, and no more than 100 ppm, the second main step of the procedure is carried out, which is the bed regeneration by the use of a liquid regeneration medium, being this a polar solvent that has a bigger affinity for isocyanuric acid than that of the adsorbent, so that isocyanuric acid passes from the adsorbent to the liquid substance and the adsorbent remains clean for a next adsorption cycle.

Preferably, the polar solvent used in the bed regeneration cycle is selected from the group which comprises: HCl, NaOH, KOH or a polar hydrocarbon selected from the group which comprises: acetone, diethylether, dimethyl formamide, dimethyl sulfoxide, methanol, ethanol and pyridine, or their mixtures. In a preferred embodiment of this invention, methanol is used.

In other preferred embodiment of this invention, the polar hydrocarbon is mixed with water in a proportion of water between 50% (vol./vol.) and 10% (vol./vol.), preferably in a proportion of 10% (vol./vol.).

In other preferred embodiment of this invention, the mixture of the polar hydrocarbon with water has pH between 4 and 10, and more preferably pH is 8.

pH is regulated by the addition of NaOH or KOH. As examples, the final balanced concentrations for a mixture of 50% water/methanol of 15 ml in contact with 1 g of saturated carbon with isocyanuric acid, are of 475, 485, 533, 470 and 490 mg/L for pHs of 2,63, 4,25, 8, 10, 11,5 respectively.

In the stage (d), it is collected the liquid regeneration medium that obtains a high concentration of isocyanuric acid in solution and its recovery is carried out in the third step of the procedure, by means of the addition of a complexing substance of the isocyanuric acid. This substance in the liquid extraction medium reacts with isocyanuric acid forming crystals of isocyanurate of microscopic size.

As a complexing substance it can be used any molecule capable of forming multiple hydrogen bridges and with some symmetry. Preferably, the complexing substance of isocyanuric acid used in the stage (d) is selected from the group which comprises: melamine, amelide, ameline, bis-diaminotriazines, tris-diaminotriazines. In a preferred embodiment of this invention, melamine is used.

In the stage (e), it is obtained a suspension of crystals, in a preferred embodiment crystals of melamine isocyanurate are obtained that, in an optional stage, is mixed with a filtering adjuvant with a particle size similar or a little bigger than that of the crystals obtained.

In a preferred embodiment, so as to get a suspension of crystals it is added a mixture of the solid complexing substance with the filtering adjuvant to the liquid regeneration medium which contains a high concentration of isocyanuric acid in solution in the third step of the procedure. An approximately 50% mixture can be used.

Preferably, the filtering adjuvant used in the stage (e) is selected from the group: natural diatomaceous earth, calcined o calcined to flow, pearlite, cellulose or any of their mixtures.

In the stage (f), the mixture obtained in the stage (e) is passed through a filtering medium of adequate mesh size for the retention of the solid mixture obtained.

Preferably, the filtering medium used has the permeability of the adjuvant between 0,04 Darcys and 10 Darcys, preferably 4 Darcys.

In the stage (g), on one hand, it is recovered the solid mixture of isocyanurate and adjuvant, if this is used, and on the other, the regenerated liquid extraction medium.

In the stage (h), the regenerated liquid extraction medium is returned to the system for the next bed regeneration cycle.

As a result of the procedure according to this invention, the solid mixture can be used as raw material for the manufacture of trichloroisocyanuric acid and the regenerated liquid extraction medium can be used in a new extraction cycle.

According to another aspect of this invention, it is disclosed a system to implement the procedure described above. For this, the system for the extraction of isocyanuric acid in solution comprises:
- a B01, pump which in the adsorption cycle, drives water from the source of origin through flow regulation means to a C01 adsorption column where isocyanuric acid is retained,
- flow regulation means which allow the isocyanuric acid free water to be returned to the source of origin,
- a D01 liquid regeneration medium deposit, connected to a D02 dosage tank in which a solid complexing substance is added, and in an optional embodiment, together with a filtering adjuvant. In this optional embodiment, an approximately 50% mixture can be used.
- Flow regulation means which allow the liquid regeneration medium to pass from the D01 deposit to the D02 dosage tank through other flow regulation means,
- a pump which drives the regeneration liquid coming from the D01 deposit through flow regulation means to the D02 dosage tank through other flow regulation means,
- A F01 solid mixture retention filter,
- a dosing pump B02, which in the regeneration cycle, doses in line the D02 deposit suspension to the F01 filter,
- Flow regulation means which allow the supply of the liquid medium coming from the D02 dosage tank to the F01 filter,
- Flow regulation means which allow to supply the regeneration liquid recovered from the F01 filter to the C01 adsorption column in the opposite direction to the adsorption cycle,
- Flow regulation means which return the regeneration liquid after passing through the C01 adsorption column to the D01 deposit.

In this way, it is obtained equipment specifically designed to keep the isocyanuric acid level in pool water within certain pre-set safe levels. The equipment is also designed to operate in automatic cycles requiring very little attention from the operator.

The equipment consists of an adsorption column with a specific adsorbent, a deposit of regenerating substance of the adsorbent, a filter for the filtration of the regenerating substance, a tank with agitation containing a filtering substance to be dosed to the regenerating substance, a compressor, a drive pump and a valve system to configure the equipment in accordance to the phase of the cycle in which it has to operate. The equipment operates in three cycles: adsorption, regeneration and rinse which will operate sequentially and in that same order.

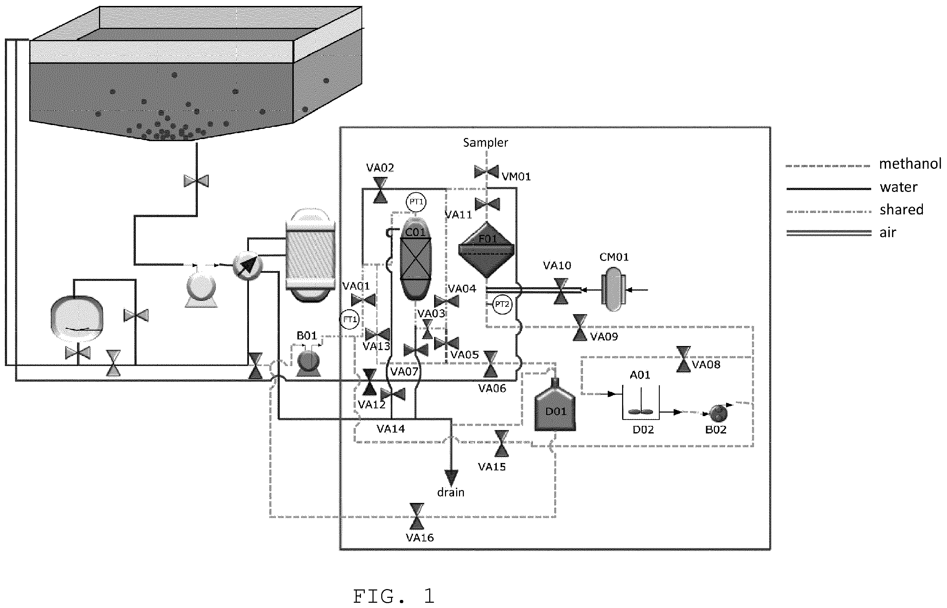

In Fig. 1 it is shown the system in accordance to the first preferred embodiment of this invention.

Specifically, the model comprises the parts enclosed in box of figure 1. According to this invention, it operates in three successive cycles: adsorption, regeneration and rinse.

The system for the extraction of isocyanuric acid in solution in accordance to this preferred embodiment comprises:
- a B01 pump, which in the adsorption cycle, drives water from the source of origin through a VA01 valve to a C01 adsorption column where isocyanuric acid is retained,
- Valves VA03, VA04 and VA12 so as to return the isocyanuric acid free water to the source of origin,
- A D01 liquid regeneration medium deposit, connected to a D02 dosage tank in which a solid complexing substance is added, preferably together with a filtering adjuvant,
- A VA16 valve which allows the passage of the liquid regeneration medium from the D01 deposit to the D02 dosage tank through VA08 and VA15 valves,
- a B01 pump which drives the regeneration liquid coming from the D01 deposit through the VA16 valve to the D02 dosage tank through the VA08 and VA15 valves,
- VA08 and VA15 valves
- A F01 solid mixture retention filter,
- a B02 dosing pump, which in the regeneration cycle, doses in line the suspension of the D02 deposit to the F01 filter,
- a VA09 valve which allows the supply of the liquid medium coming from the D02 dosage tank to the F01 filter,
- valves VA11, VA04 and VA03 so as to supply the regeneration liquid recovered from the F01 filter to the C01 adsorption column in the opposite direction to the adsorption cycle,
- Valves VA13 and VA06 which return the regeneration liquid after passing through the C01 adsorption column to the D01 deposit.

In the adsorption cycle a B01 pump drives water from the source of origin, preferably being this a swimming pool, through a VA01 valve to a C01 adsorption column where isocyanuric acid is retained by means of a specific adsorbent. This valve can be controlled in accordance to any of the ways known in the technique as for example hydraulically, pneumatically, electrically, electronically, but the use of another way of controlling is also contemplated, as for example manually. Then, water leaves the C01 column already free of isocyanuric acid and returns to the swimming pool passing through VA03, VA04 and VA12 valves. During this cycle only the mentioned valves remain open. The water returning to the swimming pool with no isocyanuric acid makes the concentration of this compound decrease progressively in the swimming pool. When the consigned safety concentration is reached the equipment prepares to finish the adsorption cycle and move on to the next cycle.

For this, it is activated the CM01 compressor which drives air through the VA10 and VA02 valves. The water evacuated from the C01 column after the adsorption cycle, is returned to the swimming pool through the VA03, VA04 and VA12 valves and, once it is empty, the CM01 compressor continues to blow for a time that can be programmed in the equipment, so as to remove as much water as possible from the C01 column. During this part of the cycle the rest of the valves remain closed. After emptying, network water is passed through the C01 column so as to rinse by passing the water through the VA07 valve and evacuating down the drain by means of the VA14 valve of the installation. The rinse time can be programmed in the equipment control box.

Once the rinse of the C01 column is finished, the regeneration cycle is started by activating the B01 pump which drives the regeneration liquid coming from the D01 deposit through the VA16 valve to the D02 dosage tank through the VA08 and VA15 valves.

In the D02 dosage tank it is added a solid powder substance, complexing of isocyanuric acid, which is suspended in the regeneration liquid with the help of the A01 agitator. At this moment it is also possible to add simultaneously to the D02 dosage tank the filtering adjuvant. Once the D02 deposit is full the VA08 valve is closed and VA09 valve is opened, reaching the F01 filter, and the VA11, VA4, VA03 valves passing through the C01 column in opposite direction to the adsorption cycle. The regeneration liquid extracts isocyanuric acid from the adsorbent, leaves the C01 column and passes through the VA13 and VA06 valves returning to the D01 deposit. Simultaneously to this process, the B02 dosing pump injects the suspension of the D02 deposit in the upstream pipe section of VA08, so that the regeneration liquid with dissolved isocyanuric acid is mixed with the solid complexing liquid in suspension remaining both retained in the F01 filter. In this way, the regeneration liquid comes out clean from the F01 filter and goes back through the C01 column to continue extracting isocyanuric acid from the adsorbent. This process continues until no more isocyanuric acid is extracted from the adsorbent of the C01 column, moment in which the regeneration cycle ends and the rinse cycle starts so as to prepare the C01 column for the adsorption filter. At this moment the C01 column is emptied by passing air of the CM01 compressor through the VA10 and VA02 valves evacuating the regeneration liquid from the C01 column after the regeneration cycle and returning it to the D01 deposit through the VA03, VA05 and VA06 valves. After the evacuation, the CM1 compressor keeps working for a time that can be programmed in the equipment, to remove as much regeneration liquid as possible from the C01 column. During this part of the cycle the rest of the valves remain closed. After emptying network water is passed through the C01 column to rinse by passing the water through the VA07 valve, evacuating this water by means of the VA14 valve and down the drain of the installation. The rinse time can be programmed in the equipment control box, being recommendable between 20 and 30 min. Once the C01 column rinse is finished, the equipment is ready to start a new adsorption cycle.

The system also incorporates a simple valve VM01, for the isocyanuric acid control at the outlet of the C01 column. If its concentration reaches a predetermined value, for example, 15ppm, the replacement of the adsorbent must be carried out in a system maintenance job.

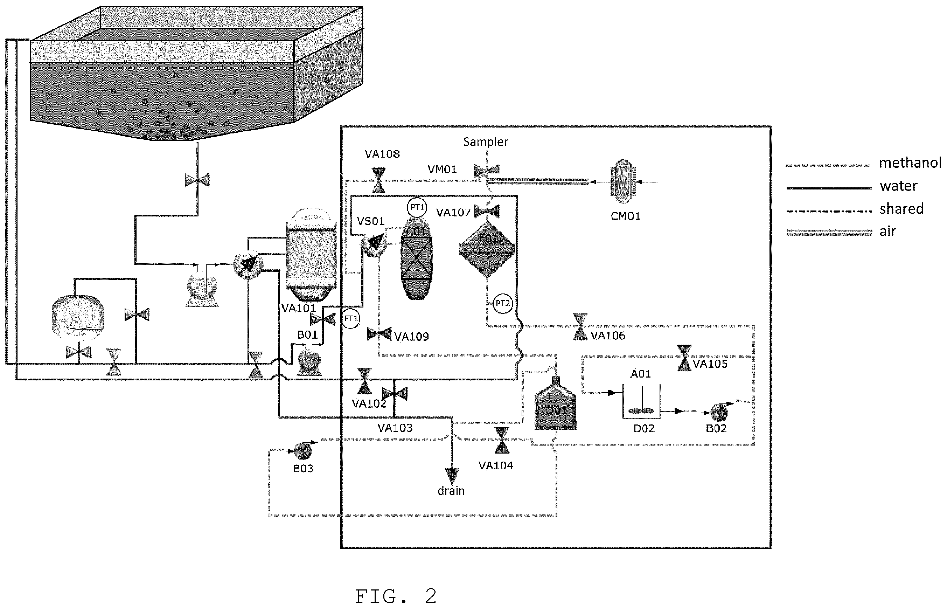

In Fig. 2 the system is shown according to the second preferred embodiment of this invention.

Specifically, the model consists of the parts enclosed in box of figure 2. According to this invention, three successive cycles are operated: adsorption, regeneration and rinse, same as in the first preferred embodiment of this invention.

The system for the extraction of isocyanuric acid in solution in accordance to this preferred embodiment comprises:
- a B01 pump, which in the adsorption cycle, drives the water from the source of origin through a VA01 valve to a selector valve VS01 and towards a adsorption column C01, where isocyanuric acid is retained,
- VS01 and VA102 valves to return the isocyanuric acid free water to the source of origin,
- a D01 regeneration liquid medium deposit, connected to a D02 dosage tank in which a solid complexing substance is added, preferably together with a filtering adjuvant,
- a VA104 valve which allows the regeneration liquid medium to pass from the D01 deposit to the D02 dosage tank through a VA105 valve,
- a B03 pump which drives the regeneration liquid coming from the D01 deposit through the VA104 valve to the D02 dosage tank through the VA105 valve,
- a F01 solid mixture retention filter,
- a B02 dosing pump, which in the regeneration cycle, drives the suspension of the D02 deposit to the F01 filter,
- a VA106 valve which allows the supply of the liquid medium coming from the D02 dosage tank to the F01 filter,
- a VA107, VA108 valve to supply the regeneration liquid recovered from the F01 filter through the VS01 selector valve to the C01 adsorption column in opposite direction to the adsorption cycle,
- VS01 and VA109 valves which return the regeneration liquid after passing through the C01 adsorption column to the D01 deposit.

In this variant of the equipment, the C01 column can be operated by a 5-way valve, being this the VS01 selector valve which simplifies the management of the system, by decreasing the number of valves used. The expert in the field will understand that it is also possible to use way-valves with another type of configurations as for example 6-way, 7-way, ...etc. valves.

In the adsorption cycle, the pool water is driven by means of the B01 pump through the VA101 valve to the VS01 selector valve and it passes through it to the C01 adsorption column, where isocyanuric acid is removed by means of a specific adsorbent. The VA101 valve can be a actuated valve, but this valve can be controlled according to any of the modes known in the technique as for example hydraulically, pneumatically, electrically, electronically. It is also contemplated the use of other ways of controlling, as for example manually. Then, the water comes out from the C01 column already free of isocyanuric acid and returns to the pool by passing through the VS01 and VA102 valves. During this cycle only these valves remain open. The water returning to the pool with no isocyanuric acid makes the concentration of this compound in the pool decrease progressively. When the consigned safety concentration is reached, the equipment prepares to finish the adsorption cycle and move on to the next cycle. For this, it is activated the CM01 compressor which drives air through the VA108 valve. The water evacuated from the C01 column is returned to the pool through the VS01 and VA102 valves and, once empty, the CM01 compressor continues to blow for a time which can be programmed in the equipment, so as to remove as much water as possible from the C01 column. During this part of the cycle the rest of the valves remain closed. After emptying, network water is passed through the C01 column to rinse by passing water through the VS01, VA103 valves, evacuating this water down the drain of the installation. The rinse time can be programmed in the equipment control box. Once the rinse of the C01 column is finished, the regeneration cycle is started activating the B03 pump which drives the regeneration liquid coming from the D01 deposit through the VA104 valve to the D02 dosage tank through the VA105 valve. In the D02 dosage tank a powder solid substance is added, complexing of isocyanuric acid, which is suspended with the help of the A01 agitator in the regeneration liquid. Once the D02 deposit is full the VA105 valve is closed and VA106 is opened, reaching the F01 filter, and the VA107, VA108 valves passing through the VS01 selector valve to the C01 column in opposite direction to the adsorption cycle. The regeneration liquid extracts isocyanuric acid from the adsorbent, leaves the C01 column and passes through the VS01 and VA09 valves, returning to the D01 deposit. Simultaneously to this process, the B02 dosing pump, injects the suspension of the D02 deposit in the upstream pipe section of the VA105 valve, so that the regeneration liquid with dissolved isocyanuric acid is mixed with the solid complexing and the adjuvant in suspension being both retained in the F01 filter. In this way, the regeneration liquid comes out clean from the F01 filter and goes back through the C01 column so as to continue extracting isocyanuric acid from the adsorbent. This process continues until no more isocyanuric acid is obtained from the adsorbent of the C01 column, moment in which the regeneration cycle ends and the rinse cycle starts to prepare the C01 column for the adsorption cycle. At this moment the emptying of the C01 column is carried out by passing air from the CM1 compressor through the VA108 and VS01 valves evacuating the regeneration liquid from the C01 column and returning it to the D01 deposit through the VA109 valve. After evacuating, the CM1 compressor keeps working for a time that can be programmed in the equipment, to remove as much regeneration liquid as possible from the C01 column. During this part of the cycle the rest of the valves remain closed. After emptying network water is passed through the C01 column to rinse passing the water through the VA103 valve, evacuating this water down the drain of the installation. The rinse time can be programmed in the equipment control box, being recommendable between 20 and 30 min. Once the rinse of the C01 column is finished, the equipment is ready to start a new adsorption cycle.

In another variant of the equipment, manual valves can be used instead of actuated ones. The expert in the field will understand that these valves can be controlled according to any of the modes known in the technique as for example hydraulically, pneumatically, electrically, electronically.

### Example of embodiment of the invention

In a first example, a solution of 10 L of 35 mg/L of isocyanuric acid was used by the addition of 625 mg of trichloroisocyanuric acid in 10 L of water. As an adsorbent an activated carbon treated with nitric acid was used to increase the affinity for the isocyanuric acid. The amount of adsorbent used was 120 ml and was placed in a column of 4,5 cm in diameter x 7 cm in height. The solution was passed through the column at a flow rate of 1,5 m/h obtaining a concentration below the detection limit of the isocyanuric acid measurement equipment for the 10 L of solution. This experiment was repeated several times so as to saturate the column and up to 95 L were passed before starting to detect isocyanuric acid at the exit of it. Water kept being passed until the exit concentration equaled the entrance one, 215 L were passed in total. Then, the column was emptied and the regeneration of it was carried out by passing 600 ml methanol firstly through the isocyanuric acid saturated carbon bed and then, and in the same process, through a ceramic filter by dosing in line a 1:1 mixture of melamine and diatom. The isocyanuric acid content analysis at the exit of the adsorption column gave a value over the detection limit of the measurement equipment at the beginning of the regeneration process and the analysis at the exit of the filter gave a value below the detection limit. The process was maintained until the isocyanuric acid content at the exit of the adsorption column was below the detection limit of the measurement equipment, which indicated the maximum extraction of the column. After the column regeneration process, this was washed with running water to eliminate remains of methanol and the adsorption process was started again until saturation. Up to 210 L of solution of isocyanuric acid were passed until the saturation of the column indicating regeneration over 97%.

In a preferred embodiment, in the system in accordance to this invention, the C01 adsorption column contains an adsorbent material selected from the group of materials in the form of porous carbon, preferably the porous carbon is selected from the group that comprises activated carbon, carbon airgel, carbon xerogel, activated carbon fibers, ordered mesoporous carbon (OMC) or graphene gels.

In another preferred embodiment, in the system in accordance to this invention, the liquid regeneration medium is a polar solvent that has a higher affinity for isocyanuric acid than that of the adsorbent, preferably the polar solvent is selected from the group that comprises: HCl, NaOH, KOH or a polar hydrocarbon selected from the group that comprises: acetone, diethylether, dimethyl formamide, dimethyl sulfoxide, methanol, ethanol and pyridine, or their mixtures.

In another preferred embodiment, in the system in accordance to this invention, the solid complexing substance of isocyanuric acid is selected from the group that comprises: melamine, amelide, ameline, bis-diaminotriazines, tris-diaminotriazines.

In another preferred embodiment, in the system in accordance to this invention, the filtering adjuvant is selected from the group: natural diatomaceous earth, calcined or calcined to flow, perlite, cellulose or any of their mixtures.

In another preferred embodiment, in the system in accordance to this invention, the F01 solid mixtureretention filter has the permeability of the diatom between 0,04 Darcys and 10 Darcys, preferably 4 Darcys.

As previously stated, the equipment developed, in accordance to this invention, consists of a adsorption column with a specific adsorbent, a deposit of regenerating substance of the adsorbent, a filter for the filtration of the regenerating substance, a deposit with agitation containing a filtering substance to dose to the regenerating substance, a compressor, a drive pump and a valves system or other flow regulation means to configure the equipment according to the phase of the cycle in which it has to operate. The expert in the field will understand that flow regulation means can be several valves or other similar means, which will be used according to the needs of each particular installation. The function of these means is to provide the connection among the elements of this invention. They can be referred to in general terms as: water flow regulation means between the source of origin and the adsorption column, flow regulation means between the liquid regeneration medium deposit and the dosage tank, flow regulation means between the dosage tank and the retention filter, means for the flow regulation of the regeneration liquid recovered from the retention filter and the adsorption column, or flow regulation means which return the regeneration liquid after passing through the adsorption column to the liquid regeneration medium deposit.

Although this invention has previously been described with reference to certain specific embodiment examples thereof, the expert in the technique will be able to conceive modifications and variations of this embodiment without thereby departing from the scope of this invention.

## Claims

1. Procedure for the extraction of isocyanuric acid in solution that comprises the following stages:
(a) The water containing isocyanuric acid is passed through a bed with an adsorbent material,
(b) The water passing through the bed is constantly returned to the source of origin,
(c) Once the determined isocyanuric acid level in the source of origin has been reached, the bed regeneration is carried out by using a liquid regeneration medium, being this a polar solvent that has a higher affinity for isocyanuric acid than that of the adsorbent,
(d) It is collected the liquid regeneration medium which contains a concentration of isocyanuric acid in solution and its recovery is carried out by adding a isocyanuric acid complexing substance,
(e) A crystals suspension is obtained, which is mixed with a filtering adjuvant of particle size bigger than the crystals obtained,
(f) The mixture obtained in the stage (e) is passed through a filtering medium of adequate mesh size for the retention of the solid mixture obtained,
(g) The solid mixture of isocyanurate and adjuvant, and the regenerated liquid extraction medium are recovered,
(h) The regenerated liquid extraction medium is returned to the system for the next bed regeneration cycle.

2. Procedure according to claim 1, **characterized in that** the adsorbent material used in the stage (a) is selected from the group of materials in the form of porous carbon.

3. Procedure according to claim 2, **characterized in that** the porous carbon is selected from the group that comprises activated carbon, carbon airgel, carbon xerogel, activated carbon fibers, ordered mesoporous carbon(OMC) or graphene gels.

4. Procedure according to claim 3, **characterized in that** activated carbon is used

5. Procedure according to any of the preceding claims, **characterized in that** the polar solvent used in the stage (c) is selected from the group that comprises: HCl, NaOH, KOH or a polar hydrocarbon selected from the group that comprises: acetone, diethylether, dimethyl formamide, dimethyl sulfoxide, methanol, ethanol and piridine, or their mixtures.

6. Procedure according to claim 5, **characterized in that** the polar solvent is methanol.

7. Procedure according to any of the claims 5 or 6, **characterized in that** the polar hydrocarbon is mixed with water in a proportion of water between 50% (vol./vol.) and 10% (vol./vol.), preferably in a proportion of 10% (vol./vol.).

8. Procedure according to claim 7, **characterized in that** the mixture has pH between 4 and 10, and preferably pH is 8.

9. Procedure according to any of the preceding claims, **characterized in that** the solid substance complexing of isocyanuric acid used in the stage (d) is selected from the group that comprises: melamine, amelide, ameline, bis-diaminotriazines, tris-diaminotriazines.

10. Procedure according to claim 9, **characterized in that** melamine is used.

11. Procedure according to any of the preceding claims, **characterized in that** the suspension of crystals of the stage (e), is mixed with a filtering adjuvant of similar or bigger particles size than the crystals obtained.

12. Procedure according to claim 11, **characterized in that** the filtering adjuvant used in the stage (e) is selected from the group: natural diatomaceous earth, calcined or calcined to flow, perlite, cellulose or any of their mixtures.

13. Procedure according to any of the preceding claims, **characterized in that** the filtering medium of the stage (f) has the permeability of the adjuvant between 0,04 Darcys and 10 Darcys, preferably 4 Darcys.

14. System for the extraction of isocyanuric acid in solution which comprises:
- a B01 pump, which, in the adsorption cycle, drives the water from the source of origin through flow regulation means to a C01 adsorption column where isocyanuric acid is retained,
- flow regulation means which allow the isocyanuric acid free water to be returned to the source of origin,
- a D01 liquid regeneration medium deposit, connected to a D02 dosage tank in which a solid complexing substance is added,
- flow regulation means which allow the liquid regeneration medium to pass from the D01 deposit to the D02 dosage tank through other flow regulation means,
- a pump which drives the regeneration liquid coming from the D01 deposit through flow regulation means to the D02 dosage tank through other flow regulation means,
- a F01 filter of retention of the solid mixture,
- a B02 dosing pump, which in the regeneration cycle, doses in line the suspension from the D02 deposit to the F01 filter,
- flow regulation means which allow the supply of the liquid medium coming from the D02 dosage tank to the F01 filter,
- flow regulation means which allow to supply the regeneration liquid recovered from the F01 filter to the C01 adsorption column in the opposite direction to the adsorption cycle,
- flow regulation means which return the regeneration liquid after passing through the C01 adsorption column to the D01 deposit.

15. System according to claim 14, **characterized in that** the solid complexing substance is added, together with a filtering adjuvant to the D02 dosage tank.

16. System for the extraction of isocyanuric acid in solution according to claim 14, **characterized in that** it comprises:
- a B01 pump, which in the adsorption cycle, drives the water from the source of origin through a VA01 valve (actuated) to a C01 adsorption column where isocyanuric acid is retained,
- VA03, VA04 and VA12 valves to return the isocyanuric acid free water to the source of origin,
- a D01 liquid regeneration medium deposit, connected to a D02 dosage tank in which a solid complexing substance is added,
- a VA16 valve which allows the liquid regeneration medium to pass from the D01 deposit to the D02 dosage tank through VA08 and VA15 valves,
- a B01 pump which drives the regeneration liquid coming from the D01 deposit through the VA16 valve to the D02 dosage tank through the VA08 and VA15 valves,
- a F01 filter of retention of the solid mixture,
- a B02 dosing pump, which in the regeneration cycle, doses in line the suspension from the D02 deposit to the F01 filter,
- a VA09 valve which allows the supply of the liquid medium coming from the D02 dosage tank to the F01 filter,
- VA11, VA04 and VA03 valves to supply the regeneration liquid recovered from the F01 filter to the C01 adsorption column in the opposite direction to the adsorption cycle,
- VA13 and VA06 valves which return the regeneration liquid after passing through the C01 adsorption column to the D01 deposit.

17. System according to claim 16, **characterized in that** the solid complexing substance is added, together with a filtering adjuvant to the D02 dosage tank.

18. System according any of the claims 16 or 17, **characterized in that** it comprises a CM01 compressor to drive the air through the VA10 and VA02 valves and evacuate the water from the C01 adsorption column after the adsorption cycle, where the evacuated water is returned to the source of origin through the VA03, VA04 and VA12 valves.

19. System according to any of the claims 16 to 18, **characterized in that** it comprises a VA07 valve to rinse the C01 adsorption column with water.

20. System according to any of the claims 16 to 19, **characterized in that** it comprises a A01 agitator to make easy the suspension in the D02 dosage tank.

21. System according to any of the claims 16 to 20, **characterized in that** the CM01 compressor drives air through the VA10 and VA02 valves evacuating the regeneration liquid from the C01 adsorption column after the regeneration cycle, where the regeneration liquid is returned to the D01 deposit through the VA03, VA05 and VA06 valves.

22. System for the extraction of isocyanuric acid in solution according to claim 14, **characterized in that** it comprises:
- a B01 pump, which in the adsorption cycle, drives the water from the source of origin through a VA101 valve to the VS01 selector valve and towards a C01 adsorption column where isocyanuric acid is retained,
- VS01 and VA102 valves to return the isocyanuric acid free water to the source of origin,
- a D01 liquid regeneration medium deposit, connected to a D02 dosage tank in which a solid complexing substance is added,
- a VA104 valve which allows the liquid regeneration medium to pass from the D01 deposit to the D02 dosage tank through a VA105 valve,
- a B03 pump which drives the regeneration liquid coming from the D01 deposit through the VA104 valve to the D02 dosage tank through the VA105 valve,
- a F01 filter of retention of the solid mixture,
- a B02 dosing pump, which in the regeneration cycle, drives the suspension from the D02 deposit to the F01 filter,
- a VA106 valve which allows to supply the liquid medium coming from the D02 dosage tank to the F01 filter,
- VA107, VA108 valves to supply the regeneration liquid recovered from the F01 filter through the VS01 connector valve to the C01 adsorption column in the opposite direction to the adsorption cycle,
- VS01 and VA109 valves which return the regeneration liquid after passing through the C01 adsorption column to the D01 deposit.

23. System according to claim 22, **characterized in that** the solid complexing substance is added, together with a filtering adjuvant, to the D02 dosage tank.

24. System according to any of the claims 22 to 23, **characterized in that** it comprises a CM01 compressor to drive air through the VA108 valve and to evacuate the water from the C01 adsorption column after the adsorption cycle, where the evacuated water is returned to the source of origin through the VS01 and VA102 valves.

25. System according to any of the claims 22 to 24, **characterized in that** it comprises the VS01 valve and a VA103 valve to rinse the C01 adsorption column with water.

26. System according to any of the claims 22 to 25, **characterized in that** it comprises a A01 agitator to make easy the suspension in the D02 dosage tank.

27. System according to any of the claims 22 to 26, **characterized in that** the CM01 compressor drives air through the VA108 and VS01 valves evacuating the regeneration liquid from the C01 adsorption column after the regeneration cycle, where the regeneration liquid is returned to the D01 deposit through a VA109 valve.

28. System according to any of the claims 14-27, **characterized in that** the C01 adsorption column contains an adsorbent material selected from the group of materials in the form of porous carbon.

29. System according to claim 28, **characterized in that** the porous carbon is selected from the group that comprises activated carbon, carbon airgel, carbon xerogel, activated carbon fibers, ordered mesoporous carbon (OMC) or graphene gels.

30. System according to any of the claims 14-29, **characterized in that** the liquid regeneration medium is a polar solvent that has a bigger affinity for isocyanuric acid than that of the adsorbent.

31. System according to claim 30, **characterized in that** the polar solvent is selected from the group that comprises: HCl, NaOH, KOH or a polar hydrocarbon selected from the group that comprises: acetone, diethylether, dimethyl formamide, dimethyl sulfoxide, methanol, ethanol and piridine, or their mixtures.

32. System according to any of the claims 14-32, **characterized in that** the solid complexing substance of isocyanuric acid is selected from the group that comprises: melamine, amelide, ameline, bis-diaminotriazines, tris-diaminotriazines.

33. System according to any of the claims 15, 17 and 23, **characterized in that** the filtering adjuvant is selected from the group: natural diatomaceous earth, calcined or calcined to flow, perlite, cellulose or any of their mixtures

34. System according to any of the claims 14-33, **characterized in that** the F01 filter of retention of the solid mixture has the permeability of diatom between 0,04 Darcys and 10 Darcys, preferably 4 Darcys.
